# EUROPEAN PATENT SPECIFICATION

(11) **EP 1 424 048 B1**
(45) Date of publication and mention of the grant of the patent: **07.03.2007**
(21) Application number: 03027333.8
(22) Date of filing: 26.11.2003
(51) Int. Cl.: A61F 2/16

(54) **Intraocular lens injection instrument**
Instrument zur Injektion einer Intraokularlinse
Instrument pour l'injection d'une lentille intraoculaire

(30) Priority: 26.11.2002 JP 2002341683
(43) Date of publication of application: 02.06.2004
(73) Proprietor: Nidek Co., Ltd., Gamagori-shi, Aichi-ken 443-0035 (JP); Takashima Sangyo Corp., Suwa-shi, Nagano-ken 392-0007 (JP)
(72) Inventor: Endo, Chiaki, Suwa-shi Nagano-ken 392-0131 (JP); Oda, Haruo, Gamagori-shi Aichi-ken 443-0011 (JP)
(74) Representative: Hofer, Dorothea

(56) References cited:
- EP-A- 0 937 443
- EP-A- 1 332 731
- EP-A- 1 415 619
- US-A- 3 831 601
- US-A- 6 059 791

## Description

### BACKGROUND OF THE INVENTION

### 1. Field of the Invention

The present invention relates to an instrument for injecting an intraocular lens into an eye of a patient.

### 2. Description of Related Art

From EP 0 937 443 A2 a device for implantation of an intraocular lens is known which comprises a drive mechanism for a plunger which is contained in a housing. The plunger's axial forward thrust movement is transferred to the intraocular lens positioned on the housing end during implantation. A piston acts as an energy accumulator on the plunger with pressure applied by a spring in the forward thrust direction, and with a damping medium applying pressure in the opposite direction to the forward thrust direction.

EP-A-1 415 619, a document which falls under the provisions of Art. 54(3) EPC, refers to an O-ring which is deformed by the application of washers.

As one of operative treatments for cataract, generally used is a method of removing a crystalline lens from an eye of a patient and then injecting an intraocular lens in place of the crystalline lens. To inject the intraocular lens, the following steps are taken: first making an incision in an eyeball of a patient's eye; fragmenting and aspirating a clouded crystalline lens through the incision by for example an ultrasonic cataract-surgery device (a phaco-emulsification device); and then injecting the intraocular lens into the eye through the incision to implant it in place of the crystalline lens.

If a large incision is made, it may become a burden on the eyeball and also cause astigmatism of the patient's eye after the operation. To prevent such the disadvantages, an intraocular lens injecting instrument called an injector is used to inject a foldable intraocular lens into an eye through a smaller incision. In this injector, the foldable intraocular lens held in a housing of the injector is pushed toward the tip of the injector while being folded into a smaller shape. Thereafter, the intraocular lens is pushed out of the tip of the injector inserted in the eye through the incision and is spread (unfolded) in the eye.

Working pressure of the above injector to push out the intraocular lens (i.e., a force needed to operate a push-out device) is fixed. Thus, the injector may often be hard to operate because some operators feel such fixed working pressure as too light (too low) or others feel it as too heavy (too high).

### SUMMARY OF THE INVENTION

The present invention has been made in view of the above circumstances and has an object to overcome the above problems and to provide an intraocular lens injection instrument of which working pressure to push out an intraocular lens can be adjusted.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

Such objects are achieved in accordance with an intraocular lens injection instrument comprising the features of claim 1.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1 is a schematic perspective view of an intraocular lens injection instrument in an embodiment;
Fig. 2 is a schematic perspective view of an insertion tube;
Fig. 3 is a sectional view of a part of an outer cylinder element in which a push-out device, a pressure member, and a flange member are attached;
Fig. 4 is an exploded view of the pressure member and the flange member;
Fig. 5 is a schematic perspective back view of a part of the injection instrument;
Fig. 6 is a schematic perspective view of a rotation angle adjustment jig;
Figs. 7A and 7B are sectional views of a part of the outer cylinder element with the push-out device, the pressure member, and the flange member, showing steps of adjusting working pressure; and
Fig. 8 shows another example of a mechanism for changing a rotation angle of a pressure member.

### DETAILED DESCRIPTION OF THE PREFERRED EMBODIMENTS

A detailed description of a preferred embodiment of an intraocular lens injection instrument embodying the present invention will now be given referring to the accompanying drawings.

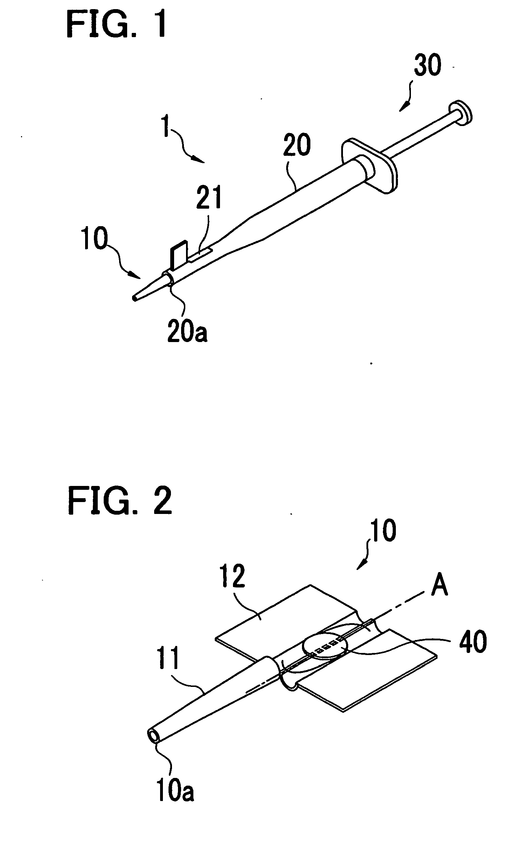

Fig. 1 is a schematic perspective view of an intraocular lens injection instrument in the present embodiment; and Fig. 2 is a schematic perspective view of an insertion tube whose lens-placing part is opened.

Numeral 1 is an injection instrument which includes, in order of insertion into an eye, an insertion tube 10 which holds an intraocular lens 40 (see Fig. 2) and is inserted in the eye through an incision formed therein, an outer cylinder element 20 having a tip end 20a in which the insertion tube 10 is mounted, and a push-out device 30 for pushing out the intraocular lens 40 through a tip end 10a of the insertion tube 10 mounted in the outer cylinder element 20. It is to be noted that the insertion tube 10 may be united with the outer cylinder element 20.

As shown in Fig. 2, the insertion tube 10 is integrally constructed of a tapered insertion part 11 whose diameter becomes smaller toward the tip end 10a and a placing part 12 in which the intraocular lens 40 is placed in a folded state. The insertion part 11 is of a hollow cylindrical shape so that the folded intraocular lens 40 is moved through the hollow portion and discharged out through the tip end 10a. The placing part 12 is configured to be openable and closable about a dashed line A. The intraocular lens 40 is placed in the placing part 12 in an opened state and then folded when the placing part 12 is closed. The intraocular lens to be used may be any one of existing foldable intraocular lenses. The structures of the insertion part 11 and the placing part 12 are well known in the art and therefore the detailed explanations thereof are here omitted.

The outer cylinder element 20 is formed, near the tip end 20a, with an opening 21 for mounting therein the insertion tube 10. The insertion tube 10 with the placing part 12 closed is put and mounted in the outer cylinder element 20 through the opening 21.

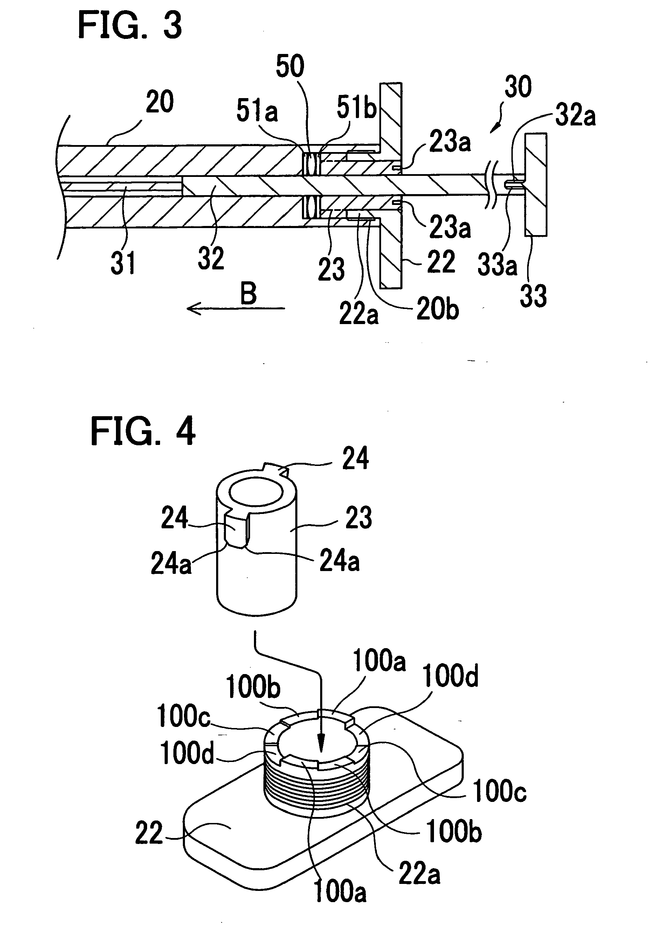

Fig. 3 is a sectional view of a part of the outer cylinder element 20 in which the push-out device 30, a pressure member 23, and a flange member 22 are attached. The outer cylinder element 20 is of a hollow cylindrical shape in which the push-out device 30 is mounted to be movable (slidable) in an axial direction of the outer cylinder element 20. The push-out device 30 is constructed of a push portion (push rod) 31, a shaft 32, and a push member 33. The push portion 31 is fixed to a tip end of the shaft 32, i.e., closer to the tip end 10a when the push-out device 30 is mounted in the outer cylinder element 20. When the shaft 32 is moved or slid frontward (in the direction of an arrow B in Fig. 3), the push portion 31 pushes the intraocular lens 40 out of the insertion tube 10 through the tip end 10a. The push member 33 is formed with a male screw portion 33a which engages with a female screw portion 32a formed in a back end of the shaft 32. Thus, the push member 33 is secured to the shaft 32. By push of this push member 33 with fingers, the operator can move or slide the shaft 32 and the push portion 31 frontward.

Numeral 22 is the flange member. Numeral 23 is the pressure member having a hollow cylindrical shape. This pressure member 23 is mounted on the push-out device 30 (the shaft 32) and besides fit in the flange member 22. The outer cylinder element 20, on a back end (an opposite end to the tip end 20a), is formed with a concave portion having a predetermined depth. This concave portion includes an inner wall surface formed with a female screw portion 20b. The flange member 22 is formed with a tubular male screw portion 22a which engages with the female screw portion 20b to screw the flange member 22 in the outer cylinder element 20. In this state where the flange member 22 is secured to the outer cylinder element 20, the pressure member 23 is held between the outer cylinder element 20 and the flange member 22 to be rotatable about the shaft 32. It is to be noted that numeral 23a is a hole (two holes in the present embodiment) formed on a back end face (a right end face in Fig. 3) of the pressure member 23. This hole 23a is used to change the rotation angle of the pressure member 23, i.e., to rotate the pressure member 23, with the use of a rotation angle adjustment jig 60 mentioned later.

As shown in Fig. 3, the flange member 22 is secured to the outer cylinder element 20, so that a clearance is formed between the concave portion of the outer cylinder element 20 and the pressure member 23. In this clearance, an O-ring 50 which is an elastic member such as rubber and washers 51a and 51b are mounted on the shaft 32. The O-ring 50 is designed to have an inside diameter equal to or slightly smaller than the diameter of the shaft 32 so that the inside circumferential surface of the O-ring 50 is always in contact with the shaft 32. It is to be noted that the elastic member is the O-ring in the present embodiment, but it is not limited thereto. The elastic member may be any member that is able to be compressed (deformed) when pressed by the pressure member 23, thereby adjusting working pressure.

The detailed constructions of the pressure member 23 and the flange member 22 are explained below with reference to Fig. 4. In the flange member 22, the male screw portion 22a is formed with a stepped tip end face (an upper end face in Fig. 4) including step portions 100a-100d which are different in height (length from a back end face of the flange member 22), including four height variations in the embodiment (a pair of step portions for each height). The paired step portions 100a and 100a, 100b and 100b... are arranged symmetrically about the center axis of the flange member 22 (the male screw portion 22a). The step portions 100a are the highest and the step portions 100b, 100c, and 100d are lower in this order.

The pressure member 23 on the outer peripheral wall near a tip end (an upper end in Fig. 4, namely, an end closer to the tip end 10a when the pressure member 23 is mounted in the outer cylinder element 20) is formed with a pair of protrusions 24 positioned symmetrically about the center axis of the pressure member 23. When the pressure member 23 is fit in the flange member 22, the back end faces (the lower end faces in Fig. 4) of the protrusions 24 contact a corresponding one pair of the step portions 100a-100d and hence the pressure member 23 is held protruding from the male screw portion 22a of the flange member 22. In this state where the pressure member 23 is fit in the flange member 22, the rotation angle of the pressure member 23 about the shaft 32 can be changed, thereby changing the total length between the back end face of the flange member 22 and the tip end face of the pressure member 23. In the present embodiment, the total length can be changed in four steps by the four height variations of the step portions 100a-100d.

It is to be noted that each protrusion 24 is formed, at back (lower in Fig. 4) corners, with chamfered portions 24a to allow smooth changing of the rotation angle of the pressure member 23. Furthermore, the height differences among the step portions 100a-100d are so determined as not to interfere with the changing of the rotation angle of the pressure member 23, preferably, determined at 0.1 mm or less, and more preferably, at about 0.05 mm or less. In the present embodiment, each height difference among the step portions 100a-100d is set at 0.1 mm, except for the height difference between the adjacent step portions 100a and 100d, but it is not limited thereto. The height difference may be any value if only the working pressure of the push-out device 30 can be adjusted and the rotation angle of the pressure member 23 can be changed without trouble.

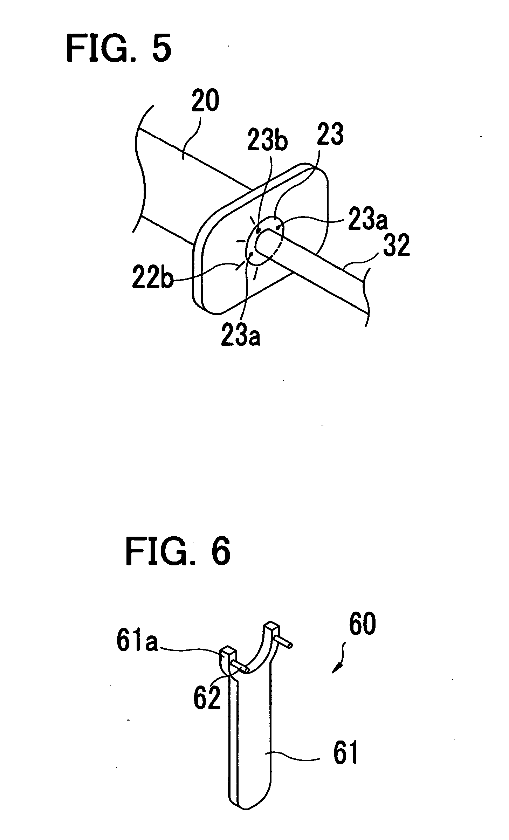

Fig. 5 is a schematic perspective back view of a part of the injection instrument 1. As shown in Fig. 5, the flange member 22 is provided, on the back end face, with scales 22b corresponding to the step portions 100a-100d to indicate working pressure levels. The pressure member 23 is provided on the back end face with a mark 23b indicating the current rotation angle (position) of the pressure member 23 (i.e., the current working pressure level). On the other hand, Fig. 6 is a schematic perspective view of a rotation angle adjustment jig 60. This jig 60 is constructed of a handle 61 formed with a Y-shaped end 61a having an inside curved surface corresponding to the curved periphery of the shaft 32, and insertion rods 62 each of which has a predetermined length and extends at right angle to the side surface of the Y-shaped end 61a. The adjustment jig 60, with the insertion rods 62 inserted in the holes 23a shown in Fig. 5, is rotated to change the rotation angle of the pressure member 23 about the shaft 32.

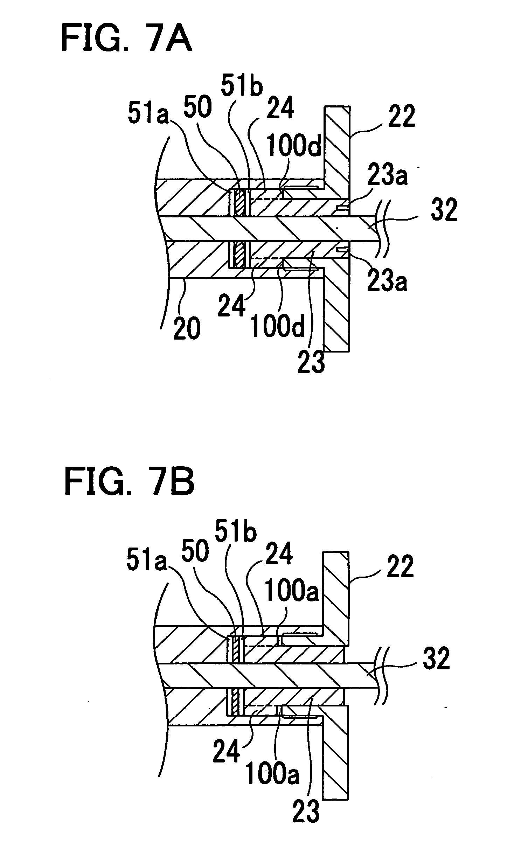

The following explanation is made, referring to Figs. 7A and 7B, on the steps of adjusting the working pressure of the intraocular lens injection instrument having the above structure.

Fig. 7A shows a sectional view of a part of the outer cylinder element 20 to which the flange member 22 is secured in the state that the protrusions 24 of the pressure member 23 are in contact with the step portions 100d of the male screw portion 22a of the flange member 22. In this state, the total length between the back end face of the flange member 22 and the tip end face of the pressure member 23 is minimum. Accordingly, the tip end face of the pressure member 23 slightly presses the O-ring 50 through the washer 51b in the axial direction.

When pressed by the pressure member 23, the O-ring 50 is compressed into a slightly flattened state within the clearance. As a result of this compression, the contact area and contact pressure of the O-ring 50 with the shaft 32 are increased, raising the frictional force therebetween. Thus, the working pressure needed to move the push-out device 30 in the axial direction can be increased.

To provide maximum working pressure, the pressure member 23 is rotated with the use of the adjustment jig 60 by a predetermined rotation angle to bring the protrusions 24 into contact with the step portions 100a. Thus, the pressure member 23 is made to slide toward the O-ring 50 by a distance corresponding to the height difference. In this state, the total length between the back end face of the flange member 22 and the tip end face of the pressure member 23 is maximum (as shown in Fig. 7B). When the total length between the back end face of the flange member 22 and the tip end face of the pressure member 23 is increased as above, the pressure member 23 further presses the O-ring 50 in the axial direction by an increased pressing force. The O-ring 50, when further pressed in the axial direction, is further compressed as compared with the state shown in Fig. 7A, thus increasing the contact area and contact pressure with respect to the shaft 32 and hence raising the frictional force. This makes it possible to increase the working pressure needed to move the push-out device 30.

To reduce the working pressure, on the other hand, the pressure member 23 is rotated with the use of the adjustment jig 60 by a predetermined rotation angle to bring the protrusions 24 into contact with an appropriate pair of the step portions 100b-100d. These step portions 100b, 100c, and 100d, in this order, can produce lower working pressure. Accordingly, the working pressure needed to move the push-out device 30 can be simply adjusted.

After adjustment of the working pressure, the operator mounts the insertion tube 10, in which the intraocular lens 40 is set in place, in the outer cylinder element 20, and then he moves the push-out device 30 under the working pressure of his own choice to push out the intraocular lens 40 into the patient's eye.

It is to be noted that, although the mechanism for adjusting the working pressure in four steps is explained as above, the number of adjusting steps may be determined as needed.

In the present embodiment, the tip end face of the male screw portion 22a of the flange member 22 is stepped to provide the step portions 100a-100d of four different heights, but it is not limited thereto. Other structures capable of adjusting the working pressure by changing the rotation angle of the pressure member 23 may also be adopted. For instance, the male screw portion 22a may be provided with a sloped tip end surface. In this case, a recess is formed in the sloped tip end surface and each protrusion 24 of the pressure member 23 is formed with the back end face shaped to be engageable with the recess, thereby producing desired working pressure. With this structure, the pressure member 23 produces a click at each rotation angle corresponding to each desired working pressure.

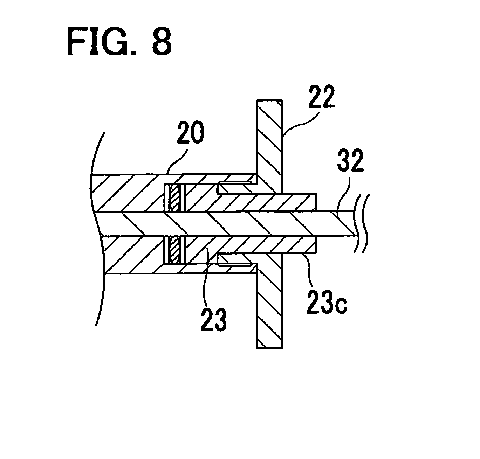

In the present embodiment, the adjustment jig 60 is used to change the rotation angle of the pressure member 23. Instead of using the jig 60, for example, a pressure member 23 may be adapted to have an extended back end protruding from the back end face of the flange member 22, as shown in Fig. 8, so that this protruding portion is used as a rotation angle adjustment part 23c. In this case, to change the rotation angle of the pressure member 23, the adjustment part 23c has only to be rotated. With this simple structure, the rotation angle of the pressure member 23 can be changed without the use of a jig.

As described above, according to the present invention, in pushing out the intraocular lens into the eye by the use of the intraocular lens injection instrument, the user (the operator) can adjust the working pressure of the injection instrument to the working pressure of his own choice.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument, including:
a cylinder (10, 20) provided with an insertion part (11) which is inserted in an eye through an incision formed in the eye; and
push-out means (30) which is mounted axially movably in the cylinder to push out an intraocular lens (40) placed in the cylinder to the outside through the insertion part;
an O-ring (50) which is placed in contact with the push-out means to produce a frictional force on the push-out means; and
adjustment means (22, 23, 51a, 51b) which changes a contact pressure or contact area of the O-ring with respect to the push-out means by changing a reformed state of the O-ring to change the frictional force to be produced by the O-ring to adjust working pressure needed to move the push-out means
wherein
the adjustment means includes pressing means (22, 23) which presses the O-ring in an axial direction of the push-out means and is rotated about the axis of the push-out means to change a pressing force on the O-ring.

2. The intraocular lens injection instrument according to claim 1, wherein the pressing means is rotated about the axis of the push-out means to change an axial length of the pressing means.

## Patentansprüche

1. Einbringungsinstrument einer intraokularen Linse mit: einem Zylinder (10, 20), der mit einem Einführungsteil (11) versehen ist, das in ein Auge durch einen im dem Auge gebildeten Einschnitt eingeführt wird; und
einem Ausstoßmittel (30), das axial bewegbar in dem Zylinder angebracht ist, zum Ausstoßen einer intraokularen Linse (40), die in dem Zylinder plaziert ist, zu der Außenseite durch das Einführungsteil;
einem O-Ring (50), der in Kontakt mit dem Ausstoßmittel plaziert ist, zum Erzeugen einer Reibungskraft auf dem Ausstoßmittel; und
einem Einstellmittel (22, 23, 51a, 51b), das einen Kontaktdruck oder eine Kontaktfläche des O-Ringes in Bezug auf das Ausstoßmittel ändert durch Ändern eines verformten Zustandes des O-Ringes zum Ändern der Reibungskraft, die durch den O-Ring zu erzeugen ist, zum Einstellen von Arbeitsdruck, der zum Bewegen des Ausstoßmittels benötigt wird;
worin das Einstellmittel ein Preßmittel (22, 23) enthält, das den O-Ring in einer axialen Richtung des Ausstoßmittels preßt und um die Achse des Ausstoßmittels zum Ändern einer Preßkraft auf dem O-Ring gedreht wird.

2. Einbringungsinstrument einer intraokularen Linse nach Anspruch 1, bei dem das Preßmittel um die Achse des Ausstoßmittels gedreht wird zum Ändern einer axialen Länge des Preßmittels.

## Revendications

1. Instrument d'injection de lentille intraoculaire, incluant :
un cylindre (10, 20) prévu avec une partie d'insertion (11) qui est insérée dans un oeil à travers une incision formée dans l'oeil ; et
un moyen de poussée (30) qui est monté axialement de façon mobile dans le cylindre pour pousser une lentille intraoculaire (40) placée dans le cylindre vers l'extérieur à travers la partie d'insertion ;
un joint torique (50) qui est placé en contact avec le moyen de poussée pour produire une force de frottement sur le moyen de poussée ; et
un moyen d'ajustement (22, 23, 51a, 51b) qui change une pression de contact ou une superficie de contact du joint torique par rapport au moyen de poussée en changeant un état déformé du joint torique pour changer la force de frottement devant être produite par le joint torique pour ajuster la pression de travail nécessaire pour déplacer le moyen de poussée,
dans lequel
le moyen d'ajustement inclut un moyen de pression (22, 23) qui presse le joint torique dans une direction axiale du moyen de poussée et est tourné autour de l'axe du moyen de poussée pour changer une force de pression sur le joint torique.

2. Instrument d'injection de lentille intraoculaire selon la revendication 1, dans lequel le moyen de pression est tourné autour de l'axe du moyen de poussée pour changer une longueur axiale du moyen de pression.
